(19) Europäisches Patentamt
European Patent Office
Office européen des brevets

(11) EP 4 061 321 B1

(12) **EUROPEAN PATENT SPECIFICATION**

(45) Date of publication and mention of the grant of the patent:
**27.09.2023 Bulletin 2023/39**

(21) Application number: **20800610.6**

(22) Date of filing: **29.10.2020**

(51) International Patent Classification (IPC):
***A61K 8/44*** *(2006.01)* ***A61K 8/86*** *(2006.01)*
***A61K 8/46*** *(2006.01)* ***A61Q 5/00*** *(2006.01)*
***A61K 8/19*** *(2006.01)* ***A61K 8/49*** *(2006.01)*
***A61Q 5/02*** *(2006.01)* ***A61K 8/73*** *(2006.01)*

(52) Cooperative Patent Classification (CPC):
**A61K 8/463; A61K 8/44; A61K 8/4926; A61K 8/4946; A61K 8/731; A61K 8/737; A61Q 5/006; A61Q 5/02**

(86) International application number:
**PCT/EP2020/080443**

(87) International publication number:
**WO 2021/099088 (27.05.2021 Gazette 2021/21)**

(54) **HAIR CARE COMPOSITION**

HAARPFLEGEZUSAMMENSETZUNG

COMPOSITION DE SOINS CAPILLAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.11.2019 PCT/CN2019/119551**
**17.12.2019 EP 19216904**

(43) Date of publication of application:
**28.09.2022 Bulletin 2022/39**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **Unilever Global IP Limited**
  **Wirral, Merseyside CH62 4ZD (GB)**
  Designated Contracting States:
  **CY DE GB IE IT MT RS TR**

(72) Inventors:
- **LIU, Jian**
  **Shanghai, 200335 (CN)**
- **PI, Yingying**
  **Shanghai, 200335 (CN)**

(74) Representative: **Mathai, Neenu Grace**
**Unilever Patent Group**
**Bronland 14**
**6708 WH Wageningen (NL)**

(56) References cited:
**WO-A1-2012/022553 WO-A2-2010/127924**
**WO-A2-2011/107468 CN-A- 110 327 282**
**US-A- 4 867 971**

- **DATABASE GNPD [Online] MINTEL; 14 January 2019 (2019-01-14), anonymous: "5D Charm Bright Shampoo", XP055691647, retrieved from www.gnpd.com Database accession no. 6260291**
- **DATABASE GNPD [Online] MINTEL; 1 June 2018 (2018-06-01), anonymous: "Chinese Honeylocust Anti-Dandruff Shampoo", XP055691656, retrieved from www.gnpd.com Database accession no. 5721175**
- **DATABASE GNPD [Online] MINTEL; 28 March 2019 (2019-03-28), anonymous: "Scalp Purifying + Bamboo Charcoal Shampoo", XP055691653, retrieved from www.gnpd.com Database accession no. 6433611**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

## Description

### Technical Field of the Invention

The present invention relates to a hair care composition, especially a hair care composition comprising an amino acid surfactant, a specific anionic surfactant and anti-dandruff agents that provides enhanced deposition of anti-dandruff agents onto hair and/or scalp of an individual.

### Background of the Invention

Hair care compositions are formulated for specific purposes, such as cleansing or conditioning benefits or a combination of the two. Preferred hair care composition will provide more benefits than simple cleansing and conditioning. For example, there is a growing demand for hair care compositions that are mild and also have an anti-dandruff benefit.

Dandruff is an issue that affects many people globally. The condition is manifested by the shedding of clumps of dead skin cells from the scalp. These are white in colour and provide an aesthetically displeasing appearance. A factor that contributes to dandruff is certain members of the Malassezia yeasts. To combat these, anti-dandruff products have included certain anti-dandruff agents which have anti-fungal activity, for example, piroctone olamine (Octopirox®), azole based anti-fungal agents (e.g. climbazole, ketoconazole), selenium sulfide or combinations thereof. Many anti-dandruff hair care compositions, however, do not provide sufficient anti-dandruff agent deposition during the excessive rinsing process. The anti-dandruff agents are simply rinsed away and therefore provide little or no anti-dandruff benefit. Therefore, it is always desired to efficiently deposit anti-dandruff agents onto hair and/or scalp to provide good anti-dandruff performance.

Anionic surfactants such as sulfated surfactants are typically incorporated into hair care compositions for their superior cleansing and foaming properties. However, sulfated surfactants like sodium lauryl sulfate or sodium lauryl ether sulfate (SLES) tend to be harsh on the skin and may adversely affect the deposition of anti-dandruff agents. One approach for providing mildness is to use mild anionic surfactants. Among such mild anionic surfactants that can be used are N-acyl amino acids and their salts. such acyl amino acids are contemplated as co-surfactants to alleviate harshness of primary anionic surfactants as seen in US patent 6,703,427.

WO2011107468 A2 discloses antidandruff formulations comprising as active agents pyrithione salts, 1-hydroxy-2-pyridone derivatives (eg. piroctone olamine) or selenium (poly)sulfides. The compositions typically comprise surfactants such as SLES.

US4867971 A discloses an antidandruff shampoo comprising climbazole and anionic surfactants (eg. SLES), and having a pH of 4 to 5. The examples show that the claimed pH range improves deposition of the antifungal agent.

WO2010127924 A2 discloses a hair treatment composition comprising a zinc based antidandruff agent, a conazole fungicide (ketoconazole or climbazole) and a cationic modified guar deposition polymer, such as guar hydroxypropyl trimonium chloride.

CN110327282 A discloses an anti-dandruff shampoo comprising cocoyl glycinate, laureth sulphate, climbazole and piroctone olamine.

It has been found unexpectedly that compositions comprising a specific combination of acyl glycinate salt and ethoxylated alkyl sulfate anionic surfactant can provide enhanced deposition of anti-dandruff agents while maintaining desirable mildness.

### Summary of the invention

In a first aspect, the present invention is directed to a hair care composition comprising:

(a) a salt of acyl glycinate;
(b) an ethoxylated alkyl sulfate anionic surfactant having a formula $R^2O(CH_2CH_2O)_nSO_3M$, wherein $R^2$ is an alkyl or alkenyl group having from 8 to 18 carbon atoms; M is a solubilising cation comprising sodium, potassium, ammonium, substituted ammonium or mixtures thereof; n is the degree of ethoxylation of from 0.5 to 3; and
(c) an anti-dandruff agent selected from piroctone olamine, azole based anti-fungal agents and mixtures thereof;
wherein the composition comprises the salt of acyl glycinate and the ethoxylated alkyl sulfate anionic surfactant in a weight ratio of from 1:10 to 1:1.

In a second aspect, the present invention is directed to a packaged hair care product comprising the hair care composition of the first aspect of this invention.

In a third aspect, the present invention is directed to a method of depositing anti-dandruff agents onto scalp comprising the step of applying the hair care composition of any embodiment of the first aspect of this invention onto

scalp surfaces of an individual.

All other aspects of the present invention will more readily become apparent upon considering the detailed description and examples which follow.

## Detailed Description

Except in the examples, or where otherwise explicitly indicated, all numbers in this description indicating amounts of material or conditions of reaction, physical properties of materials and/or use may optionally be understood as modified by the word "about".

All amounts are by weight of the final hair care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis*.

"Hair care composition", as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and rinse-off shampoos, conditioners, shower gels, or toilet bar. The composition of the present invention is preferably a rinse-off composition, especially preferred being a shampoo or a conditioner and most preferably a shampoo.

The salt of acyl glycinate is an amino acid-based surfactant derived from the amino acid glycine. Glycine is the smallest of the naturally occurring amino acids. This small size facilitates production of smaller surfactant micelles and the generation of a creamy lather during use. The salt of acyl glycinate suitable for use in compositions of the present invention has a general formula (I):

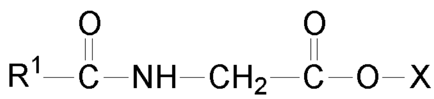

(I)

wherein $R^1$ is alkyl or alkenyl group having 7 to 23 carbons, preferably 9 to 17 carbons, and X is a solubilising cation comprising sodium, potassium, ammonium, substituted ammonium or mixtures thereof. It is preferred that the cation is sodium or potassium, more preferably sodium.

Suitable examples of the salt of acyl glycinate that may be used in this invention include, but not limited to, sodium capryloyl glycinate, sodium cocoyl glycinate, sodium lauroyl glycinate, sodium myristoyl glycinate, sodium palmitoyl glycinate, sodium stearoyl glycinate, sodium oleoyl glycinate, potassium capryloyl glycinate, potassium cocoyl glycinate, potassium lauroyl glycinate, potassium myristoyl glycinate or mixtures thereof. It is particularly preferred that the salt of acyl glycinate is sodium cocoyl glycinate, sodium lauroyl glycinate or mixtures thereof.

The salt of acyl glycinate is typically present in an amount of from 0.1 to 20% by weight of the composition, more preferably from 0.5 to 10% and most preferably from 1 to 5%, based on total weight of the hair care composition and including all ranges subsumed therein.

The hair care composition of the present invention comprises an ethoxylated anionic surfactant which is an ethoxylated alkyl sulfate anionic surfactant having a formula $R^2O(CH_2CH_2O)_nSO_3M$, wherein $R^2$ is an alkyl or alkenyl group having from 8 to 18 (preferably 12 to 18) carbon atoms, M is a solubilising cation comprising sodium, potassium, ammonium, substituted ammonium or mixtures thereof, n is the degree of ethoxylation of from 0.5 to 3, preferably from 1 to 3. An example is sodium lauryl ether sulfate (SLES).

"Degree of Ethoxylation", as used herein, refers to the average number of moles of ethylene oxide unit per mole of ethoxylated product. The degree of ethoxylation is measured using $^1H$ NMR in a solvent of deuterium oxide ($D_2O$). For example, the degree of ethoxylation of sodium lauryl ether sulfate (SLES) is measured using $^1H$ NMR (Bruker-Biospin, 400MHz) and the spectrum is recorded at 25°C. The sample for measurement using NMR is prepared as follows: the sample is dispersed in $D_2O$ in a centrifugal tube and sonicated, then the solution is filtered and transferred to an NMR tube. The peaks corresponding to the protons of the sample appear at about 3.98 ppm, about 4.15 ppm and

between about 3.58 to about 3.84 ppm. The peaks corresponding to the protons for $-CH_2-$, which appear at about 4.15 ppm and about 3.98 ppm, are integrated as A1. The peaks corresponding to the four protons for $-OCH_2CH_2-$, which appear at 4.15 ppm and between about 3.58 to about 3.84 ppm, are also integrated as A2. The degree of ethoxylation of SLES may be calculated as follows:

$$\text{The degree of ethoxylation} = \frac{(A2/4)}{(A1/2)}$$

Preferred ethoxylated alkyl sulfate anionic surfactant is sodium lauryl ether sulfate (SLES) having a degree of ethoxylation of from 0.5 to 3, preferably from 1 to 3.

The ethoxylated alkyl sulfate anionic surfactant is typically present in an amount of from 0.1 to 45% by weight of the hair care composition, more preferably from 1 to 30% and most preferably from 5 to 20%, based on total weight of the hair care composition and including all ranges subsumed therein.

The pH of the hair care composition is preferably equal or higher than 4.0, more preferably from 4.0 to 7.0, more preferably still from 4.0 to 6.0, and most preferably from 4.0 to 5.0. The salt of acyl glycinate is difficult to solubilize at lower pH ranges because the molecule will tend to precipitate. To formulate a homogeneous composition with good flowability, it is preferable if the amount of acyl glycinate salts in the composition is equal or lower than the amount of ethoxylated alkyl sulfate anionic surfactant. The composition of the present invention comprises the salt of acyl glycinate and the ethoxylated alkyl sulfate anionic surfactant in a weight ratio of from 1:10 to 1:1, preferably from 1:8 to 1:1 and more preferably from 1:6 to 1:1.5, most preferably from 1:5.5 to 1:2.25, including all ratios subsumed therein.

The hair care composition also comprises anti-dandruff agents, which are compounds that are active against dandruff and are typically anti-microbial agents and preferably anti-fungal agents. Suitable anti-dandruff agents that may be used in this invention are selected from piroctone olamine (Octopirox®), azole based anti-fungal agents and mixtures thereof.

Without wishing to be bound by theory the present inventors believe that the composition of the present invention can work for the deposition of piroctone olamine or azole based anti-fungal agents because both of them can be stabilized and solved in micelles, and the deposition can be improved by reducing the active's solubility in the system.

The preferred azole based anti-fungal agent is ketoconazole, climbazole or mixtures thereof. Preferably, the anti-dandruff agent is selected from piroctone olamine, climbazole and mixtures thereof. In an especially preferred embodiment, the anti-dandruff agent is piroctone olamine.

The hair care composition of the invention typically comprises the anti-dandruff agent in an amount of from 0.01 to 10%, more preferably from 0.01 to 5%, more preferably still from 0.05 to 2%, and most preferably from 0.05 to 1.5%, based on total weight of the hair care composition and including all ranges subsumed therein.

In addition to the salt of acyl glycinate and the ethoxylated alkyl sulfate anionic surfactant, it is preferred if the hair care composition also comprises other surfactants.

Examples of suitable anionic surfactants include alkyl sulfates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule. Typical examples of anionic surfactants include, but not limited to, sodium oleyl succinate, ammonium lauryl sulphosuccinate, sodium lauryl sulfate, sodium lauryl ether sulphosuccinate, ammonium lauryl sulfate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate. Mixtures of any of the foregoing anionic surfactants may also be suitable. Generally, the total amount of additional anionic surfactant in hair care composition of the present invention ranges from 0.5 to 45%, more preferably from 1.5 to 35% and most preferably from 5 to 20%, based on total weight of the hair care composition and including all ranges subsumed therein.

The hair care composition may also comprise non-ionic surfactants, which can be included in an amount of from 0.1 to 10%, preferably from 0.5 to 8%, more preferably from 1 to 5%, based on total weight of the hair care composition and including all ranges subsumed therein. Examples of suitable non-ionic surfactants include condensation products of aliphatic ($C_8$ - $C_{18}$) primary or secondary linear or branched chain alcohols or phenols with alkylene oxides, usually ethylene oxide and generally having from 6 to 30 ethylene oxide groups. Alkyl ethoxylates are preferred, more preferably are alkyl ethoxylates having the formula $R^3$-$(OCH_2CH_2)_mOH$, where $R^3$ is an alkyl chain having from 12 to 15 carbon atoms and m is from 5 to 9.

Other suitable non-ionic surfactants include mono- or di-alkyl alkanolamides. Examples include coco mono- or di-ethanolamide and coco mono-isopropanolamide.

Further non-ionic surfactants which can be included in compositions of the invention are the alkyl polyglycosides (APGs). Typically, APG is one which comprises an alkyl group connected (optionally via a bridging group) to a block of one or more glycosyl groups. Preferred APGs are defined by the following formula:

$$R^4O\text{-}(G)_k$$

wherein $R^4$ is a branched or straight chain alkyl group which may be saturated or unsaturated and G is a saccharide group. $R^4$ may represent a mean alkyl chain length of from $C_5$ to $C_{20}$. Preferably $R^4$ represents a mean alkyl chain length of from $C_8$ to $C_{12}$. Most preferably the value of $R^4$ lies between 9.5 and 10.5. G may be selected from $C_5$ or $C_6$ monosaccharide residues and is preferably a glucoside. G may be selected from the group comprising glucose, xylose, lactose, fructose, mannose and derivatives thereof. Preferably G is glucose. The degree of polymerisation, k, may have a value of from 1 to 10 or more; preferably, the value of k lies from 1.1 to 2; most preferably the value of m lies from 1.3 to 1.5. Suitable alkyl polyglycosides for use in the invention are commercially available and include for example those materials identified as: Oramix NS10 ex Seppic; Plantaren 1200 and Plantaren 2000 ex Henkel.

The hair care composition may also comprise amphoteric or zwitterionic surfactants, which can be included in an amount of from 0.1 to 10%, preferably from 0.5 to 8%, more preferably from 1 to 5%, based on total weight of the hair care composition and including all ranges subsumed therein. Examples include alkyl amine oxides, alkyl betaines, alkyl amidopropyl betaines, alkyl sulphobetaines (sultaines), alkyl amphoacetates, alkyl amphopropionates, alkyl amidopropyl hydroxysultaines, wherein the alkyl group has from 8 to 19 carbon atoms. Preferably, the co-surfactant is a betaine surfactant. Typical amphoteric and zwitterionic surfactants for use in the invention include lauryl amine oxide, cocodimethyl sulphopropyl betaine, lauryl betaine, cocamidopropyl betaine and sodium cocoamphoacetate. Cocamidopropyl betaine (CAPB) is particularly preferred.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The composition may further comprise a cationic polymer. Suitable cationic polymers may be homopolymers or be formed from two or more types of monomers. The molecular weight of the polymer will generally be between 5,000 and 10,000,000 g/mol, typically at least 10,000 g/mol and preferably from 100,000 to 2,000,000 g/mol.

The polymers will have cationic nitrogen containing groups such as quaternary ammonium or protonated amino groups, or a mixture thereof. The cationic nitrogen containing group will generally be present as a substituent on a fraction of the total monomer units of the cationic polymer. Thus when the polymer is not a homopolymer it can contain spacer non-cationic monomer units. The ratio of the cationic to non-cationic monomer units is selected to give a polymer having a cationic charge density in the required range.

Suitable cationic polymers include, for example, copolymers of vinyl monomers having cationic amine or quaternary ammonium functionalities with water soluble spacer monomers such as (meth)acrylamide, alkyl and dialkyl (meth) acrylamides, alkyl (meth)acrylate, vinyl caprolactone and vinyl pyrrolidine. The alkyl and dialkyl substituted monomers preferably have $C_1$-$C_7$ alkyl groups, more preferably $C_1$-$C_3$ alkyl groups. Other suitable spacers include vinyl esters, vinyl alcohol, maleic anhydride, propylene glycol and ethylene glycol.

Preferably, the cationic polymer is a cationic polysaccharide polymer such as cationic cellulose derivatives, cationic starch derivatives, and cationic guar gum derivatives. Suitably, such cationic polysaccharide polymers have a molecular weight of from 100,000 g/mol to 2,300,000 g/mol, more preferably from 150,000 g/mol to 2,000,000 g/mol. Such cationic polysaccharide polymers preferably have a cationic charge density from 0.1 to 4 meq/g.

Cationic polysaccharide polymers suitable for use in compositions of this invention include those represented by the general formula:

$$A\text{-}O\text{-}[R^5\text{-}N^+(R^6)(R^7)(R^8)X^-]$$

wherein: A is an anhydroglucose residual group, such as a starch or cellulose anhydroglucose residual. $R^5$ is an alkylene, oxyalkylene, polyoxyalkylene, or hydroxyalkylene group, or combination thereof. $R^6$, $R^7$ and $R^8$ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in $R^6$, $R^7$ and $R^8$) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418) and copolymers of etherified cellulose and starch (e.g. as described in U.S. Patent 3,958,581).

A particularly preferred type of cationic polysaccharide polymer that can be used in compositions of the present invention is a cationic guar gum derivative, such as guar hydroxypropyltrimonium chloride (for example, commercially available from Solvay in their Jaguar trademark series or from Ashland in their N-Hance trademark series). Examples of such materials are Jaguar® C-13S, Jaguar® C-14S, Jaguar® C-17, Jaguar® Excel, Jaguar® C-162, Jaguar® C-500, Jaguar® Optima, Jaguar® LS, N-Hance™ BF17, N-Hance™ BF13 and N-Hance™ CCG45.

Mixtures of any of the above cationic polymers may be used. The cationic polymer preferably comprises cationic cellulose derivatives, cationic guar gum derivatives or mixtures thereof. Guar hydroxypropyltrimonium chloride is particularly preferred.

When used, the cationic polymer will generally be present in the hair care composition of the present invention in an amount of from 0.001 to 1% by weight of the hair care composition, more preferably from 0.01 to 0.5%, and most preferably from 0.03 to 0.3%, based on total weight of the hair care composition and including all ranges subsumed therein.

The hair care composition may additionally comprise a conditioning agent to provide conditioning benefit. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. Preferably, the conditioning agent is non-volatile, meaning that it has a vapour pressure of less than 1000 Pa at 25°C.

Preferably, the hair care composition comprises discrete dispersed droplets of a water-insoluble conditioning agent, which has a mean droplet diameter ($D_{3,2}$) of less than 15 microns, preferably less than 10 microns, more preferably less than 5 microns, most preferably less than 3 microns. The mean droplet diameter ($D_{3,2}$) of a water-insoluble conditioning agent may be measured by means of a laser light scattering technique, for example using a 2600D Particle Sizer from Malvern Instruments.

The water-insoluble conditioning agent may include non-silicone conditioning agent comprising non-silicone oily or fatty materials such as hydrocarbon oils, fatty esters and mixtures thereof. Preferably, the water-insoluble conditioning agent is emulsified silicone oil.

Suitable silicones include polydiorganosiloxanes, in particular polydimethylsiloxanes which have the CTFA designation dimethicone. Also suitable for use in compositions of this invention (particularly shampoos and conditioners) are polydimethyl siloxanes having hydroxyl end groups, which have the CTFA designation dimethiconol. Also suitable for use in compositions of this invention are silicone gums having a slight degree of crosslinking, as are described for example in WO 96/31188. Preferably, the silicone oil comprises dimethicone, dimethiconol or a mixture thereof.

The viscosity of the emulsified silicone itself (not the emulsion or the final hair care composition) is typically at least 10,000 cSt (centi-Stokes=$mm^2 \cdot S^{-1}$) at 25°C, preferably at least 60,000 cSt, most preferably at least 500,000 cSt, ideally at least 1,000,000 cSt. Preferably the viscosity does not exceed $10^9$ cSt for ease of formulation. Suitable methods for measuring the kinematic viscosity of silicone oils are known to those skilled in the art, e.g. capillary viscometers. For high viscosity silicones, a constant stress rheometer can be used to measure viscosity.

Suitable emulsified silicones for use in the hair care compositions of this invention are available as pre-formed silicone emulsions from suppliers of silicones such as Dow Silicones Corporation and GE silicones. The use of such pre-formed silicone emulsion is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer.

Examples of suitable pre-formed silicone emulsions include MEM-7128, MEM-1785, MEM-1788, all available from Dow Silicones Corporation. These are emulsions of dimethiconol/dimethicone.

Another class of silicones which may be used are functionalized silicones such as amino functional silicones, meaning a silicone containing at least one primary, secondary or tertiary amine group, or a quaternary ammonium group. Examples of suitable amino functional silicones include polysiloxanes having the CTFA designation "amodimethicone."

The water-insoluble conditioning agent is generally present in hair care composition of this invention in an amount from 0.05 to 15%, preferably from 0.1 to 10%, more preferably from 0.5 to 8%, most preferably from 1 to 5%, based on total weight of the hair care composition and including all ranges subsumed therein.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as

Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

The suspending agent is generally present in hair care composition of this invention in an amount of from 0.1 to 10%, more preferably from 0.5 to 6%, and most preferably from 0.5 to 4%, based on total weight of the hair care composition and including all ranges subsumed therein.

Preservatives may also be incorporated into the hair care composition of this invention to protect against the growth of potentially harmful microorganisms. Suitable traditional preservatives include alkyl esters of parahydroxybenzoic acid, hydantoin derivatives, propionate salts, and a variety of quaternary ammonium compounds. Illustrative yet non-limiting examples of the types of preservatives that may be used in this invention include, for examples, phenoxyethanol, sodium salicylate, methyl paraben, butyl paraben, propyl paraben, diazolidinyl urea, sodium dehydroacetate, benzyl alcohol, sodium benzoate, iodopropynyl butylcarbamate, caprylyl glycol, disodium EDTA or mixtures thereof. In an especially preferred embodiment, the preservative is phenoxyethanol, sodium salicylate or a mixture thereof. Preservatives are preferably employed in amounts ranging from 0.01 to 2% by weight of the hair care composition.

The hair care composition of the present invention may contain other ingredients which are common in the art to enhance physical properties and performances. Suitable ingredients include but are not limited to fragrance, dyes and pigments, pH adjusting agents, pearlescers or opacifiers, viscosity modifiers, thickeners, and natural hair nutrients such as botanicals, fruit extracts, sugar derivatives and amino acids. Such ingredients typically and collectively make up less than 20% by weight of the composition, and preferably, from 0.0 to 15% by weight, and most preferably, from 0.01 to 12% by weight of the composition, including all ranges subsumed therein.

The compositions of the invention are primarily intended for topical application to scalp and/or at least a portion of the hair of an individual, either in rinse-off or leave-on compositions, preferably in rinse-off compositions like shampoos.

The following examples are provided to facilitate an understanding of the present invention. The examples are not provided to limit the scope of the claims.

## Examples

### Example 1

This example demonstrates the deposition of anti-dandruff agents by using a combination of acyl glycinate salt and ethoxylated alkyl sulfate anionic surfactant. Compositions were prepared according to the formulations detailed in Table 1. All ingredients are expressed by weight percent of the total formulation, and as level of active ingredient.

**Table 1**

| Ingredient | Samples | | | | |
|---|---|---|---|---|---|
| | 1 | 2 | 3 | 4 | 5 |
| Sodium lauryl ether sulfate (1EO) | 13.00 | 11.00 | 9.00 | 11.00 | 9.00 |
| Piroctone olamine | 0.50 | 0.50 | 0.50 | 0.50 | 0.50 |
| Disodium EDTA | 0.05 | 0.05 | 0.05 | 0.05 | 0.05 |
| Sodium benzoate | 0.50 | 0.50 | 0.50 | 0.50 | 0.50 |
| Carbopol 980 | 0.35 | 0.35 | 0.35 | 0.35 | 0.35 |
| Guar Hydroxypropyltrimonium chloride[a] | 0.20 | 0.20 | 0.20 | 0.20 | 0.20 |
| Dimethiconol[b] | 0.78 | 0.78 | 0.78 | 0.78 | 0.78 |
| Dimethicone[c] | 0.52 | 0.52 | 0.52 | 0.52 | 0.52 |
| Sodium cocoyl glycinate | -- | 2.00 | 4.00 | -- | -- |
| Sodium lauryl glycinate | -- | -- | -- | 2.00 | 4.00 |
| Perfume | 0.75 | 0.75 | 0.75 | 0.75 | 0.75 |

(continued)

| Ingredient | Samples | | | | |
|---|---|---|---|---|---|
| | 1 | 2 | 3 | 4 | 5 |
| Sodium chloride | 0.50 | 0.50 | 0.50 | 0.50 | 0.50 |
| Water | To 100 | To 100 | To 100 | To 100 | To 100 |
| a. Commercial guar hydroxypropyltrimonium chloride has a weight average molecular weight of from 1.0 to 1.5 million g/mol and a degree of substitution of from 0.16 to 0.20 sourced from Lamberti.<br>b. Commercial dimethiconol which has a particle size of 0.2 μm from Dow Silicones Corporation.<br>c. Commercial dimethicone which has a particle size of 10 μm from Dow Silicones Corporation. | | | | | |

*Methods*

Citric acid was added to the samples to adjust the pH to 4.3. About 0.2 grams of the test sample was taken on artificial skin (VITRO-SKIN from IMS testing group). This was diluted with 1.8 mL water and rubbed with a plastic rod for 30 seconds. The artificial skin surface was then rinsed twice with water, first time with 4 mL water for 30 second and then again with 4 mL water for 30 seconds. The deposition of piroctone olamine on the skin (10.75 $cm^2$ per plate) was measured using HPLC method.

*Results*

The average deposition (of five such experiments) are reported in Table 2 (error represents standard deviation for duplicate measurements).

**Table 2**

| Samples | 1 | 2 | 3 | 4 | 5 |
|---|---|---|---|---|---|
| Piroctone olamine deposition (μg/plate) | 5.52±0.33 | 8.14±0.35 | 9.88±0.55 | 8.01±0.53 | 9.85±0.67 |

The results showed that samples 2 to 5 comprising a combination of acyl glycinate salt and sodium lauryl ether sulfate provided significantly better ($p<0.01$) deposition of piroctone olamine than sample 1 comprising only sodium lauryl ether sulfate (used as control).

An attempt was made to formulate samples with higher amounts of acyl glycinate salts, however samples turned out to be inhomogeneous with solid precipitation and very low flowability.

Example 2

This example demonstrates the effect of different anti-dandruff agents. All ingredients are expressed by weight percent of the total formulation, and as level of active ingredient.

**Table 3**

| Ingredient | Samples | | | |
|---|---|---|---|---|
| | 6 | 7 | 8 | 9 |
| Sodium lauryl ether sulfate (1EO) | 13.00 | 11.00 | 13.00 | 11.00 |
| Piroctone olamine | 0.50 | 0.50 | -- | -- |
| Zinc pyrithione (ZPTO) | -- | -- | 0.50 | 0.50 |
| Disodium EDTA | 0.05 | 0.05 | 0.05 | 0.05 |
| Sodium benzoate | 0.50 | 0.50 | 0.50 | 0.50 |
| Carbopol 980 | 0.35 | 0.35 | 0.35 | 0.35 |
| Dimethiconol[b] | 0.78 | 0.78 | 0.78 | 0.78 |
| Dimethicone[c] | 0.52 | 0.52 | 0.52 | 0.52 |

(continued)

| Ingredient | Samples | | | |
|---|---|---|---|---|
| | 6 | 7 | 8 | 9 |
| Sodium cocoyl glycinate | -- | 2.00 | -- | 2.00 |
| Perfume | 0.75 | 0.75 | 0.75 | 0.75 |
| Sodium chloride | 0.50 | 0.50 | 0.50 | 0.50 |
| Water | To 100 | To 100 | To 100 | To 100 |

*Methods*

The same protocol was used to measure the deposition of piroctone olamine on artificial skin as described in Example 1.

*Results*

The average deposition (of five such experiments) are reported in Table 4 (error represents standard deviation for duplicate measurements).

**Table 4**

| Samples | 6 | 7 | 8 | 9 |
|---|---|---|---|---|
| Piroctone olamine deposition (μg/plate) | 6.43±0.53 | 8.48±1.02 | -- | -- |
| ZPTO deposition (μg/plate) | --- | -- | 2.17±0.20 | 2.34±0.11 |

The results showed that sample 7 comprising a combination of acyl glycinate salt and sodium lauryl ether sulfate provided significantly better ($p<0.01$) deposition of piroctone olamine compared to sample 6 which comprised only sodium lauryl ether sulfate, while sample 9 provided comparable deposition of ZPTO to sample 8.

**Example 3**

This example demonstrates the deposition of climbazole (an azole based anti-fungal agent) by using a combination of acyl glycinate salt and ethoxylated alkyl sulfate anionic surfactant. Compositions were prepared according to the formulations detailed in Table 5. All ingredients are expressed by weight percent of the total formulation, and as level of active ingredient.

**Table 5**

| Ingredient | Samples | | | |
|---|---|---|---|---|
| | 10 | 11 | 12 | 13 |
| Sodium lauryl ether sulfate (1EO) | 13.00 | 11.00 | 9.00 | 9.00 |
| Climbazole | 0.50 | 0.50 | 0.50 | 0.50 |
| Propylene Glycol | 1 | 1 | 1 | 1 |
| Disodium EDTA | 0.05 | 0.05 | 0.05 | 0.05 |
| Sodium benzoate | 0.50 | 0.50 | 0.50 | 0.50 |
| Carbopol 980 | 0.35 | 0.35 | 0.35 | 0.35 |
| Guar Hydroxypropyltrimonium chloride[a] | 0.20 | 0.20 | 0.20 | 0.20 |
| Dimethiconol[b] | 0.78 | 0.78 | 0.78 | 0.78 |
| Dimethicone[c] | 0.52 | 0.52 | 0.52 | 0.52 |
| Sodium cocoyl glycinate | -- | 2.00 | 4.00 | -- |

(continued)

| Ingredient | Samples | | | |
|---|---|---|---|---|
| | 10 | 11 | 12 | 13 |
| Sodium lauryl glycinate | -- | -- | -- | 4.00 |
| Perfume | 0.75 | 0.75 | 0.75 | 0.75 |
| Sodium chloride | 0.50 | 0.50 | 0.50 | 0.50 |
| Water | To 100 | To 100 | To 100 | To 100 |

a. Commercial guar hydroxypropyltrimonium chloride has a weight average molecular weight of from 1.0 to 1.5 million g/mol and a degree of substitution of from 0.16 to 0.20 sourced from Lamberti.
b. Commercial dimethiconol which has a particle size of 0.2 μm from Dow Silicones Corporation.
c. Commercial dimethicone which has a particle size of 10 μm from Dow Silicones Corporation.

*Methods*

The same protocol was used to measure the deposition of piroctone olamine on artificial skin as described in Example 1.

*Results*

The average deposition (of five such experiments) are reported in Table 6 (error represents standard deviation for duplicate measurements).

**Table 6**

| Samples | 10 | 11 | 12 | 13 |
|---|---|---|---|---|
| Climbazole deposition (μg/plate) | 5.97±0.79 | 7.53±0.65 | 7.68±0.73 | 8.63±1.38 |

The results showed that samples 11 to 13 comprising a combination of acyl glycinate salt and sodium lauryl ether sulfate provided significantly better ($p<0.01$) deposition of climbazole than sample 10 comprising only sodium lauryl ether sulfate (used as control).

## Claims

1. A hair care composition comprising:

   (a) a salt of acyl glycinate;
   (b) an ethoxylated alkyl sulfate anionic surfactant having a formula $R^2O(CH_2CH_2O)_nSO_3M$, wherein $R^2$ is an alkyl or alkenyl group having from 8 to 18 carbon atoms; M is a solubilising cation comprising sodium, potassium, ammonium, substituted ammonium or mixtures thereof; n is the degree of ethoxylation of from 0.5 to 3; and
   (c) an anti-dandruff agent selected from piroctone olamine, azole based anti-fungal agents and mixtures thereof; wherein the composition comprises the salt of acyl glycinate and the ethoxylated alkyl sulfate anionic surfactant in a weight ratio of from 1:10 to 1:1.

2. The hair care composition according to claim 1, wherein the salt of acyl glycinate has a structure represented by the formula (I):

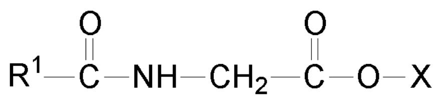

(I)

wherein $R^1$ is alkyl or alkenyl group having 8 to 22 carbons, preferably 8 to 18 carbons, and X is a solubilising cation

comprising sodium, potassium, ammonium, substituted ammonium or mixtures thereof, preferably sodium or potassium.

3. The hair care composition according to claim 1 or claim 2, wherein the salt of acyl glycinate comprises sodium capryloyl glycinate, sodium cocoyl glycinate, sodium lauroyl glycinate, sodium myristoyl glycinate, sodium palmitoyl glycinate, sodium stearoyl glycinate, sodium oleoyl glycinate, potassium capryloyl glycinate, potassium cocoyl glycinate, potassium lauroyl glycinate, potassium myristoyl glycinate or mixtures thereof, preferably sodium cocoyl glycinate, sodium lauroyl glycinate or mixtures thereof.

4. The hair care composition according to any of the preceding claims, wherein the salt of acyl glycinate is present in an amount of from 0.1 to 20%, preferably from 0.5 to 10% by weight of the composition.

5. The hair care composition according to any of the preceding claims, wherein the ethoxylated alkyl sulfate anionic surfactant is sodium lauryl ether sulfate.

6. The hair care composition according to any of the preceding claims, wherein the composition comprises the salt of acyl glycinate and the ethoxylated alkyl sulfate anionic surfactant in a weight ratio of from 1:8 to 1:1.

7. The hair care composition according to any of the preceding claims, wherein the anti-dandruff agent is selected from piroctone olamine, climbazole and mixtures thereof.

8. The hair care composition according to claim 7, wherein the anti-dandruff agent is piroctone olamine.

9. The hair care composition according to any of the preceding claims, wherein the composition comprises from 0.01 to 10% by weight of the anti-dandruff agent, preferably from 0.01 to 5%.

10. The hair care composition according to any of the preceding claims, wherein the composition additionally comprises a cationic polymer.

11. The hair care composition according to claim 10, wherein the cationic polymer comprises cationic cellulose derivatives, cationic guar gum derivatives or mixtures thereof.

12. The hair care composition according to claim 11, wherein the cationic polymer is guar hydroxypropyltrimonium chloride.

13. The hair care composition according to any of the preceding claims, wherein the composition additionally comprises a conditioning agent, preferably silicone oil, more preferably dimethicone, dimethiconol or a mixture thereof.

14. The hair care composition according to any of the preceding claims, wherein the pH of the composition ranges from 4.0 to 7.0, preferably from 4.0 to 6.0.

15. A method of depositing anti-dandruff agents onto scalp comprising the step of applying the hair care composition according to any of the preceding claims onto scalp surfaces of an individual followed by rinsing the surfaces with water.

**Patentansprüche**

1. Haarpflegezusammensetzung, umfassend:

(a) ein Salz von Acylglycinat;
(b) ein anionisches ethoxyliertes Alkylsulfat-Tensid der Formel $R^2O(CH_2CH_2O)_nSO_3M$, worin $R^2$ eine Alkyl- oder Alkenylgruppe mit 8 bis 18 Kohlenstoffatomen ist; M ein solubilisierendes Kation ist, umfassend Natrium, Kalium, Ammonium, substituiertes Ammonium oder Mischungen davon; n der Ethoxylierungsgrad von 0,5 bis 3 ist; und
(c) ein Antischuppenmittel, ausgewählt aus Piroctón-Olamin, Antimykotika auf Azolbasis und Mischungen davon; wobei die Zusammensetzung das Salz von Acylglycinat und das anionische ethoxylierte Alkylsulfat-Tensid in einem Gewichtsverhältnis von 1:10 bis 1:1 umfasst.

**2.** Haarpflegezusammensetzung nach Anspruch 1, wobei das Salz von Acylglycinat eine Struktur aufweist, dargestellt durch die Formel (I):

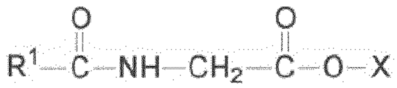 (I),

worin $R^1$ eine Alkyl- oder Alkenylgruppe mit 8 bis 22 Kohlenstoffatomen, vorzugsweise mit 8 bis 18 Kohlenstoffatomen ist und X ein solubilisierendes Kation ist, umfassend Natrium, Kalium, Ammonium, substituiertes Ammonium oder Mischungen davon, vorzugsweise Natrium oder Kalium.

**3.** Haarpflegezusammensetzung nach Anspruch 1 oder Anspruch 2, wobei das Salz von Acylglycinat umfasst Natriumcapryloylglycinat, Natriumcocoylglycinat, Natriumlauroylglycinat, Natriummyristoylglycinat, Natriumpalmitoylglycinat, Natriumstearoylglycinat, Natriumoleoylglycinat, Kaliumcapryloylglycinat, Kaliumcocoylglycinat, Kaliumlauroylglycinat, Kaliummyristoylglycinat oder Mischungen davon, vorzugsweise Natriumcocoylglycinat, Natriumlauroylglycinat oder Mischungen davon.

**4.** Haarpflegezusammensetzung nach einem der vorhergehenden Ansprüche, wobei das Salz von Acylglycinat in einer Menge von 0,1 bis 20%, vorzugsweise von 0,5 bis 10%, bezogen auf das Gewicht der Zusammensetzung, vorliegt.

**5.** Haarpflegezusammensetzung nach einem der vorhergehenden Ansprüche, wobei das anionische ethoxylierte Alkylsulfat-Tensid Natriumlaurylethersulfat ist.

**6.** Haarpflegezusammensetzung nach einem der vorhergehenden Ansprüche, wobei die Zusammensetzung das Salz von Acylglycinat und das anionische ethoxylierte Alkylsulfat-Tensid in einem Gewichtsverhältnis von 1:8 bis 1:1 umfasst.

**7.** Haarpflegezusammensetzung nach irgendeinem der vorhergehenden Ansprüche, wobei das Antischuppenmittel aus Pirocton-Olamin, Climbazol und Mischungen davon ausgewählt ist.

**8.** Haarpflegezusammensetzung nach Anspruch 7, wobei das Antischuppenmittel Pirocton-Olamin ist.

**9.** Haarpflegezusammensetzung nach einem der vorhergehenden Ansprüche, wobei die Zusammensetzung 0,01 bis 10%, bezogen auf das Gewicht des Antischuppenmittels, vorzugsweise 0,01 bis 5%, umfasst.

**10.** Haarpflegezusammensetzung nach einem der vorhergehenden Ansprüche, wobei die Zusammensetzung zusätzlich ein kationisches Polymer umfasst.

**11.** Haarpflegezusammensetzung nach Anspruch 10, wobei das kationische Polymer kationische Cellulosederivate, kationische Guargummi-Derivate oder Mischungen davon umfasst.

**12.** Haarpflegezusammensetzung nach Anspruch 11, wobei das kationische Polymer Guar-hydroxypropyltrimoniumchlorid ist.

**13.** Haarpflegezusammensetzung nach einem der vorhergehenden Ansprüche, wobei die Zusammensetzung zusätzlich ein Konditionierungsmittel, vorzugsweise Silikonöl, bevorzugter Dimethicon, Dimethiconol oder eine Mischung davon enthält.

**14.** Haarpflegezusammensetzung nach einem der vorhergehenden Ansprüche, wobei der pH-Wert der Zusammensetzung in dem Bereich von 4,0 bis 7,0, vorzugsweise von 4,0 bis 6,0, liegt.

**15.** Verfahren zum Aufbringen von Antischuppenmitteln auf die Kopfhaut, umfassend den Schritt des Auftragens der Haarpflegezusammensetzung nach einem der vorhergehenden Ansprüche auf die Kopfhautoberflächen eines Individuums, gefolgt vom Abspülen der Oberflächen mit Wasser.

**Revendications**

1. Composition pour le soin des cheveux comprenant :

(a) un sel de glycinate d'acyle ;
(b) un tensioactif anionique de sulfate d'alkyle éthoxylé ayant une formule $R^2O(CH_2CH_2O)_nSO_3M$, dans laquelle $R^2$ est un groupe alkyle ou alcényle ayant de 8 à 18 atomes de carbone ; M est un cation de solubilisation comprenant du sodium, potassium, ammonium, ammonium substitué ou des mélanges de ceux-ci ; n est le degré d'éthoxylation de 0,5 à 3 ; et
(c) un agent anti-pelliculaire choisi parmi la piroctone olamine, des agents anti-fongiques à base d'azote et des mélanges de ceux-ci ; dans laquelle la composition comprend le sel de glycinate d'acyle et le tensioactif anionique de sulfate d'alkyle éthoxylé dans un rapport de masse de 1:10 à 1:1.

2. Composition pour le soin des cheveux selon la revendication 1, dans laquelle le sel de glycinate d'acyle présente une structure représentée par la formule (I) :

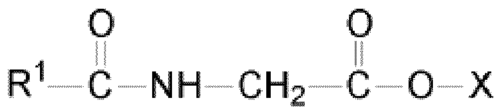

(I)

dans laquelle $R^1$ est un groupe alkyle ou alcényle ayant de 8 à 22 atomes de carbone, de préférence 8 à 18 carbones, et X est un cation de solubilisation comprenant du sodium, potassium, ammonium, ammonium substitué ou des mélanges de ceux-ci, de préférence sodium ou potassium.

3. Composition pour le soin des cheveux selon la revendication 1 ou revendication 2, dans laquelle le sel de glycinate d'acyle comprend le capryloyl glycinate de sodium, cocoyl glycinate de sodium, lauroyl glycinate de sodium, myristoyl glycinate de sodium, palmitoyl glycinate de sodium, stéaroyl glycinate de sodium, oléoyl glycinate de sodium, capryloyl glycinate de potassium, cocoyl glycinate de potassium, lauroyl glycinate de potassium, myristoyl glycinate de potassium ou mélanges de ceux-ci, de préférence cocoyl glycinate de sodium, lauroyl glycinate de sodium ou mélanges de ceux-ci.

4. Composition pour le soin des cheveux selon l'une quelconque des revendications précédentes, dans laquelle le sel de glycinate d'acyle est présent dans une quantité de 0,1 à 20 %, de préférence de 0,5 à 10 % en masse de la composition.

5. Composition pour le soin des cheveux selon l'une quelconque des revendications précédentes, dans laquelle le tensioactif anionique de sulfate d'alkyle éthoxylé est le lauryl éther sulfate de sodium.

6. Composition pour le soin des cheveux selon l'une quelconque des revendications précédentes, dans laquelle la composition comprend le sel de glycinate d'acyle et le tensioactif anionique de sulfate d'alkyle éthoxylé dans un rapport de masse de 1:8 à 1:1.

7. Composition pour le soin des cheveux selon l'une quelconque des revendications précédentes, dans laquelle l'agent anti-pelliculaire est choisi parmi la piroctone olamine, le climbazole et des mélanges de ceux-ci.

8. Composition pour le soin des cheveux selon la revendication 7, dans laquelle l'agent anti-pelliculaire est la piroctone olamine.

9. Composition pour le soin des cheveux selon l'une quelconque des revendications précédentes, dans laquelle la composition comprend de 0,01 à 10 % en masse de l'agent anti-pelliculaire, de préférence de 0,01 à 5 %.

10. Composition pour le soin des cheveux selon l'une quelconque des revendications précédentes, dans laquelle la composition comprend de plus un polymère cationique.

11. Composition pour le soin des cheveux selon la revendication 10, dans laquelle le polymère cationique comprend des dérivés de cellulose cationique, dérivés de gomme guar cationique ou des mélanges de ceux-ci.

**12.** Composition pour le soin des cheveux selon la revendication 11, dans laquelle le polymère cationique est le chlorure de guar hydroxypropyltrimonium.

**13.** Composition pour le soin des cheveux selon l'une quelconque des revendications précédentes, dans laquelle la composition comprend de plus un agent de conditionnement, de préférence de l'huile de silicone, encore mieux de la diméthicone, du diméthiconol ou un mélange de ceux-ci.

**14.** Composition pour le soin des cheveux selon l'une quelconque des revendications précédentes, dans laquelle le pH de la composition est de 4,0 à 7,0, de préférence de 4,0 à 6,0.

**15.** Procédé de dépôt d'agents anti-pelliculaires sur le cuir chevelu comprenant l'étape d'application de la composition pour le soin des cheveux selon l'une quelconque des revendications précédentes sur des surfaces de cuir chevelu d'un individu suivie par le rinçage des surfaces avec de l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6703427 B
- WO 2011107468 A2
- US 4867971 A
- WO 2010127924 A2
- CN 110327282 A
- US 3962418 A
- US 3958581 A
- WO 9631188 A